# EUROPEAN PATENT APPLICATION

(11) **EP 4 604 134 A1**
(43) Date of publication of application: **20.08.2025**
(21) Application number: 25158732.5
(22) Date of filing: 19.02.2025
(51) Int. Cl.: G16H 40/20, A61M 1/36, G16H 40/63

(54) **SYSTEM AND METHOD FOR SEPARATING WHOLE BLOOD INTO BLOOD COMPONENTS**

(30) Priority: 19.02.2024 US 202463555296 P
(71) Applicant: Fenwal, Inc., Lake Zurich, IL 60047 (US)
(72) Inventor: JONES, Kristy, Lake Zurich, 60047 (US); MEIXELSPERGER, Dale, Lake Zurich, 60047 (US); SIVERTSON, Kate, Lake Zurich, 60047 (US); VILARDI, Roberto, Lake Zurich, 60047 (US)
(74) Representative: Maikowski & Ninnemann Patentanwälte Partnerschaft mbB

(57) **Abstract**

Systems and methods for separating whole blood into blood components includes a blood component separator and a processing circuit. The processing circuit receives whole blood characteristic data and at least one of inventory data and priority data. The processing circuit identifies a blood separator process based on the whole blood characteristic data and the at least one of inventory data and priority data. The blood component separator receives a first container comprising whole blood and automatically operates or is manually operated according to the identified blood separator process to separate at least one blood component from the whole blood for collection into a second or multiple, blood component containers.

## Description

### BACKGROUND

The present application relates generally to separating whole blood into blood components. The present application relates more specifically to improved selection and/or prioritization of blood separation processes to better address supply of blood components or improve efficiencies.

Blood components, such as red blood cells, platelets, cryo and plasma, are used in transfusion facility settings to treat injury and disease or sent to fractionators for further processing. For example, red blood cell transfusions can be performed for patients suffering from anemia. Platelet transfusions can be performed to limit bleeding and hemorrhaging in patients with excessive bleeding, bleeding disorders, or hematologic malignancies. Plasma, platelets, red blood cells, and/or whole blood may be transfused during surgical procedures to replace blood or blood components that have been lost.

Blood component separators are programmable to separate whole blood in a whole blood container into different sets of manufactured blood components. For example, a unit of whole blood can be separated into plasma and red blood cells or into cryo and cryo poor plasma. Furthermore, under emergency situations or if a blood component separator is not available, blood components may be manually separated.

### BRIEF DESCRIPTION OF THE DRAWINGS

Exemplary embodiments are described below with reference to the accompanying drawings, wherein like numerals denote like elements, and:
FIG. 1 is a schematic block diagram depicting a blood processing facility having blood component separators and a computer network, according to an illustrative embodiment.
FIG. 2 is a block diagram of a system for separating whole blood into blood components, according to an illustrative embodiment.
FIG. 3 is a flow diagram showing data flows between the whole blood separator and processing circuit, according to an illustrative embodiment.
FIG. 4 is a flow diagram showing data flows between the whole blood separator, processing circuit and external computer system, according to an illustrative embodiment.
FIG. 5 is a block diagram of a processing circuit, according to an illustrative embodiment.
FIG. 6 is a display screen showing an overview of whole blood units to be processed, according to an illustrative embodiment.
FIG. 7 is a flowchart of a method of separating whole blood into blood component products, according to an illustrative embodiment.
FIG. 8 is a matrix representing stored data elements in a matrix format, according to an illustrative embodiment.

### DETAILED DESCRIPTION OF ILLUSTRATIVE EMBODIMENTS

One or more embodiments described herein may help workers select manufacturing programs that better address the needs of a facility or its customers.

One or more embodiments described herein may streamline the workflow of manufacturing blood component products.

One or more embodiments described herein may help prioritize and/or optimize the manufacturing of blood component products.

One or more embodiments described herein may improve the management of inventories of blood components.

One or more embodiments described herein may improve the predictability, efficiency, and/or automation of blood component supply chains.

One or more embodiments described herein may employ a forecasting engine which uses current inventory levels and hospital blood component demand to forecast manufacturing needs for a worker defined period of time.

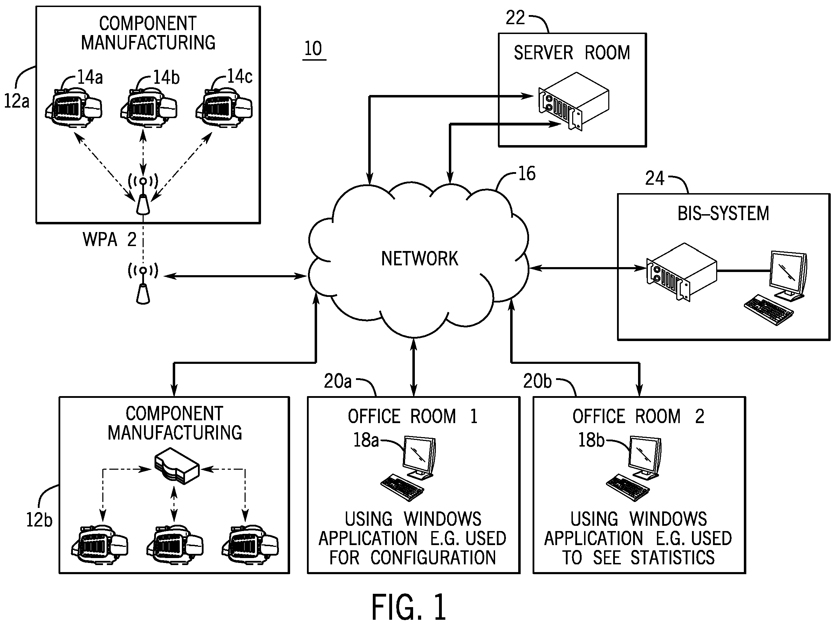

Referring to FIG. 1, a schematic block diagram shows a blood processing facility 10 having a system comprising blood component separators 14 and a computer network 16. The facility may be a manufacturing environment used by workers to process whole blood and manufacture therefrom blood component products, such as platelet products, plasma products, cryo products and red blood cell products. The facility may comprise one or more component manufacturing rooms 12a, 12b, each room containing one or more blood component separators 14a, 14b, 14c. Blood component separators may be automated, using one of a variety of electrically-powered spinners or other separators. One non-limiting example of such an automated separator is the CompoMat G5 Plus Automated Blood Component Separator manufactured by Fresenius Kabi. Blood component separators may comprise manual blood component separators, such as those using a hand operated separator or spinner, which may not have electronics features described herein or may have fewer electronics features than an automated separator. Each blood component separator may comprise a receptable configured to receive a whole blood container. The whole blood container may have been filled at a blood donation center at a location different than facility 10. Each blood component separator 14 may comprise an integrated code scanner to scan barcodes or other identifying data from the whole blood container or other items. Each blood component separator 14 may comprise an automated opener to open one or more bag breakers on a whole blood container. Each blood component separator 14 may further comprise a blood separation device, such as a centrifuge, spinning membrane, etc. configured to separate whole blood into its constituent elements. Each blood component separator 14 may further comprise a processing circuit configured to operate a plurality of different programs for separating the whole blood by way of one or more pumps or presses, clamps, detectors, etc. A user interface may be coupled to the processing circuit to facilitate operator control of the separator 14, display alerts, results and/or other data. Each blood component separator 14 may further comprise a network interface circuit configured to communicate between the on-board processing circuit and other computers over networks such as a local area network/wide local area network 16 and/or other networks.

Facility 10 may further comprise one or more client computers 18a, 18b which may be located in office rooms 20a, 20b which may be separate rooms from manufacturing rooms 12a, 12b. In some embodiments, client computers 18a, 18b may operate according to an operating system and application programs and communicate via respective network interface circuits over network 16 with blood component separators 14. Network interface circuits may be configured for communication using one or more protocols, such as REpresentational State Transfer (REST), Hypertext Transfer Protocol Secure (HTTPS), socket communication, and/or other protocols. The system includes a communication network 16 through which some or all of the various computer devices communicate with one another. In some embodiments, a plurality of the devices are configured to transmit information to and receive information from a server 22 via the communication network 16. Network 16 can comprise a local area network (LAN), a wide area network (WAN), or other networks. In some embodiments, the network is a wireless communication network to which at least some of the devices are connected, such as, for example, a mobile WiMAX network, LTE network, Wi-Fi network, or other wireless network. In other embodiments, the communication between at least some of the system computer devices and the processing circuit 22 occurs over the Internet via a wired network, such as a DSL cable connection, or over Ethernet or an intranet. In one embodiment, client computer 18a may be used by a worker to configure and/or program devices 14a-c and client computer 18b may be used by a worker to view reports, statistics, usage data, collection data, inventory data and/or other data generated by devices in facility 10. Client computers 18a, 18b may be specialized computers configured solely for connection to system 10, or they may be generalized computers made to perform specialized functions by way of programmed microprocessors. For example, in some embodiments, the various client devices 18a, 18b are desktop computers, laptop computers, and/or mobile devices such as tablets or smartphones. In some such embodiments, each client device 18a, 18b may include an internet browser, through which workers can access, and interact with, one or more devices in system 10. In various embodiments, processing circuit 22 may comprise a database server on which information sets such as inventory logs, scheduling logs, and other records are stored. Processing circuit 22 may be formed of any suitable number of servers. For example, in some embodiments, the processing circuit 22 includes one or a plurality of application servers, one or a plurality of web servers, one or a plurality of database servers, shared processing resources, etc.

Processing circuit 22 may comprise one or more analog and/or digital circuit components configured to carry out processes described herein. Processing circuit 22 may comprise a server computer, shared server resources, cloud computing, a database, and/or other computing resources. Processing circuit 22 may be configured to communicate via a network interface circuit over network(s) 16 with separators 14, client computers 18, and/or a blood bank information system 24 associated with facility 10. Blood bank information system 24 may be a Blood Establishment Computer System (BECS) or other system configured to run applications used by facility 10 to operate functions of its business, such as storing donor information, recruitment, blood component supply chain management, client relations management, manufacturing work-in-progress (WIP), and/or other functions.

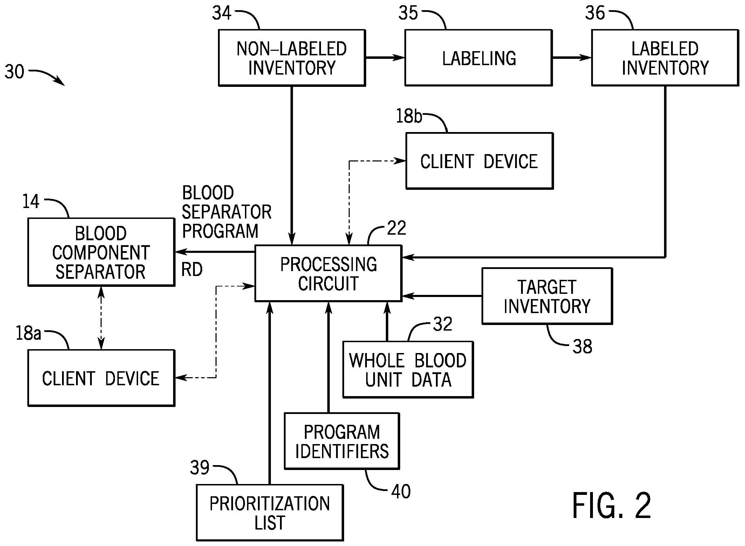

Referring now to FIG. 2, a system 30 for separating whole blood into blood components will be described according to an illustrative embodiment. Processing circuit 22 may be configured to operate one or more data management programs to carry out processes described herein. Processing circuit 22 may be configured to operate a manufacturing optimizer algorithm configured to assist workers with manufacturing blood component products from whole blood in a laboratory setting. For example, processing circuit 22 may determine, identify, or select which blood components (and/or quantities thereof) are to be manufactured from whole blood units. Processing circuit 22 may be configured to make the determination based on whole blood unit characteristics, center inventory needs, blood center goals, prioritization lists and/or other data. Processing circuit 22 may be configured to access a prioritization list 39 for data indicating priorities such as optimizing staff, ensuring blood products manufacturing timing is based on whole blood characteristics (e.g., collection time, temperature, etc.) and fulfilling stat customer orders. Processing circuit 22 may be configured to access or receive data from one or more sources (e.g., sources external to processing circuit 22, accessible over a network, or sources local to processing circuit 22), such as whole blood unit data source 32, inventory sources 34, 36, target or goal inventory source 38, program identifier source 40, client device 18b, prioritization list source 39 and/or other data sources. The data sources may comprise a database, a computer, or other electronic memory circuit configured to communicate with processing circuit 22. In some examples, the data source may be local to processing circuit 22, for example in the case where a worker uses client device 18b to configure program identifiers in a memory local to processing circuit 22. In some examples, the data source may be remote to processing circuit 22, for example in the case where whole blood unit data 32 is accessed from BIS/BECS 24 or other computing device hosting such data. In some examples, whole blood characteristic data may be received from a blood bank information system (e.g., BIS, BECS, etc.) dedicated to the blood bank, wherein the whole blood characteristic data comprises donation identifiers and/or ABO Rh blood type for a plurality of whole blood products which may have been collected from donors by the blood bank.

In one illustrative embodiment, processing circuit 22 may be configured to receive whole blood characteristic data 32 and blood center inventory data 34 and to identify a blood separator program based on the whole blood characteristic data 32, one or more of the blood center inventory data 34 and the priority list 39. Whole blood characteristic data 32 may comprise one or more of donation characteristics, donor characteristics or other whole blood characteristic data. Donation characteristics may comprise one or more of a donation identity (ID) number or other code, date and time the whole blood unit was collected, start time, stop time, duration of collection, bag type (e.g., double bag, triple bad, quad bag, etc., which may be encoded in a bag lot number or product code number, which may be scannable), temperature, such as whether the whole blood unit was transported on ice or not, amount (e.g., weight in grams, volume in mL, etc.), and/or other donation characteristic data. Donor characteristic data may comprise one or more of donor gender, donor ABO Rh blood type, antibodies present in the donor's blood, whether donor had a past pregnancy or was never pregnant, etc.

In some embodiments, processing circuit 22 may be configured to operate a balancing algorithm configured to balance data received from a plurality of the sources described in FIG. 2 and to identify a whole blood separation program as determined by the balancing algorithm. For example, the balancing algorithm may be configured to give prioritization to an inventory level (over other source data) in a case where an inventory level is not at target level for a product. In another example, the balancing algorithm may be configured to give prioritization to an urgent order request in a case where urgent order requests are above a target inventory level. In another example, the balancing algorithm may be configured to give prioritization to an inventory program in a case where a manager desires to focus on a program for a shift knowing it may take them over an inventory level. Other balancing algorithms and methods of prioritization are contemplated.

In some embodiments, a blood bank inventory module may be provided as part of BIS/BECS 24 and/or in communication therewith. A blood bank inventory module may track the inventory of a blood bank allowing the blood bank to know what supply of blood components it has in its reserves. In some embodiments, a log of the blood bank's inventory is stored in a computer database. Blood component units added to the inventory are added to the inventory log and blood component units removed from the inventory are removed from the inventory log. The inventory log includes details about the blood component units stored in the blood bank's inventory. For example, in some embodiments, the inventory log at least includes a unique identifier, the component type, the blood type, and the expiration date for each blood component unit. As a further example, an entry for a particular blood component bag may list: a number or other code uniquely identifying the bag; an indication of whether the bag contains whole blood, plasma, platelets, RBC, etc. (i.e., the component type); an indication of whether the bag contains components from A, B, AB, or O blood (i.e., the blood type); and the date at which the blood component is no longer safe or recommended for transfusion into humans (i.e., the expiration date). Additional blood component characteristics, including the date the blood component was donated, may also be included. In some embodiments, some or all such information is contained within, embedded within, or encoded within the unique blood component identifier. In some embodiments, some or all such information is contained within, embedded within, or encoded within a bar code, RFID code, or other scannable or graphical representation of data. In some embodiments, the blood components are not yet labeled and will be later within the process.

**In** one example, the blood bank inventory data may comprise a number of transfusable plasma products in inventory at the blood bank. A transfusable plasma product may be a quantity of plasma separated from a whole blood container having at least a maximum volume. The blood bank inventory data may further comprise an indication that the number of transfusable plasma products in inventory (say X units) is less than a predetermined number of transfusable plasma products (say Y units), Y units being an inventory goal or target, such as that available from target inventory data source 38. Processing circuit 22 may use this data to identify a blood separator program that includes a transfusable plasma product collection, etc., wherein at least one blood component manufactured is a transfusable plasma. Target inventory data source 38 may comprise target or goal data configurable by a worker to represent desired inventory numbers and/or amount of different manufactured blood products. Target inventory data source 38 may be external to processing circuit 22 and accessible over a network, by way of a USB drive, etc., or may be stored locally on processing circuit 22, for example by way of input via a client device. Client computer 18b may be configured to receive an inventory goal from a worker and to transmit the inventory goal to processing circuit 22, wherein processing circuit 22 is configured to identify the blood separator program based further on the inventory goal. Target inventory data may comprise prioritized target inventory levels. Prioritization list data source 39 may comprise a desired mix of transfusable plasma product versus fractionated plasma product to be manufactured and when to be manufactured (e.g., due to urgency in fulfilling a transfusable plasma product customer order, due to time consideration when whole blood data was collected and when manufacturing random donor platelets, etc.). **In** some embodiments, client computer 18b may be configured to receive a blood product type priority from a worker and to transmit the blood product type priority to the processing circuit, wherein the processing circuit is configured to identify the blood separator program based further on blood product type priority. The blood product type priority may indicate that A+ platelets have a priority 1, O+ plasma has a priority 2, and AB RBCs have a priority 3. In different embodiments, the system recognizes at least three priorities, at least five priorities, at least seven priorities, etc.

Processing circuit 22 may further be configured to receive whole blood characteristic data from whole blood unit data source 32. For example, the whole blood characteristic data may comprise a donor characteristic of the whole blood, such as donor ABO Rh blood type. The processing circuit may further identify the blood separator program based on an indication that a number or amount of blood component products of the donor ABO Rh blood type in the blood center inventory data is less than a predetermined number or amount of blood component products of the donor ABO Rh blood type. The predetermined number or amount of blood component products may represent a target inventory or goal, or minimum inventory for each of the plurality of different blood types (A+, B-, O+, AB-, etc.).

Processing circuit 22 may be configured to access a list, table or matrix of program identifiers from program identifier data source 40. Processing circuit 22 may look up a program identifier (e.g., a numeric identifier, alphanumeric code, name of program or product, etc.) associated with the identified blood separator program for use by blood component separator 14. Program identifier data source 40, like other data sources in FIG. 2, may be stored locally to processing circuit 22 and/or be accessible by processing circuit 22 over an external connection, such as a network.

Referring again to FIG. 2, blood component separator 14 may be programmed with the identified or selected blood separator program in any of a number of ways. In one example, processing circuit 22 may display an identifier of the selected blood separator program to the worker on client device 18a (e.g., a desktop computer, laptop computer, smartphone, tablet computer, general-purpose or special-purpose computer, etc.), after which the worker of client device 18a accepts or manually programs changes to the selected blood separator program which will be run on the blood component separator 14 using a user interface thereof (e.g., keypad, buttons, touch screen, etc.). In another example, processing circuit 22 may be configured to transmit an identifier of the blood separator program over a communication link to blood component separator 14 to automatically program separator 14. The processing circuit 22 may transmit the identifier in response to a request message received by processing circuit 22, wherein processing circuit 22 is configured to transmit a response message comprising the identity of the blood separator program. The request message may come from a client device usable by a worker or from a blood component separator in the case where the separator is an automated separator. In the case where the separator is a manual separator, a worker may obtain a whole blood unit ID (e.g., by scanning with a nearby code scanner, by reading from a label on the whole blood unit, or by other method) and enter the whole blood unit ID into a client device for transmission as the request message to processing circuit 22.

In the case where blood component separator 14 is a manual separator, processing circuit 22 may transmit the selected blood separator program to a client device usable by the worker, which may be in the vicinity of the manual separator. The worker can then attach a whole blood container to separator 14 and operate the identified blood separator program to separate at least one blood component from the whole blood for collection in one or more blood component containers according to the selected blood separator program.

Separator 14 may receive a separation program identifier, allowing a worker to confirm the program or to reject and then override the program. Other programming methods may be employed, such as transferring a program from processing circuit 22 to separator 14 using a universal serial bus drive or other memory card. In yet another embodiment, processing circuit 22 may be a part of blood component separator 14 and may further share processing resources therewith, such that separator 14 essentially has the capabilities of processing circuit 22 embedded therein. With blood component separator 14 programmed with the identified or selected blood separator program, a worker attaches a whole blood container to separator 14 and operates the identified blood separator program to separate at least one blood component from the whole blood for collection in one or more blood component containers.

Inventory data may comprise non-labeled inventory 34 and/or labeled inventory 36. Block 35 represents a labelling process of applying a printed label to a blood product container. Non-labeled inventory data source 34 may comprise data representing number and/or amount of a plurality of different blood products (e.g., A+ plasma, O- platelets, etc.) for blood products which have been manufactured or processed but are pending labelling at labelling process 35. Labeled inventory data source 36 may comprise data representing number and/or amount of blood products which have been labeled.

In some examples, whole blood characteristic data (e.g., comprising donation identifiers, ABO Rh blood type, and/or other data) for a plurality of whole blood products can be accessed by processing circuit 22. Processing circuit 22 may be configured to associate different blood separator programs with each of a plurality of the different donation identifiers. For example, a plasma with red blood cell program may be selected by processing circuit 22 for whole blood units 0123 and 4416, while a platelet with plasma program may be selected by processing circuit 22 for whole blood unit 4230. Processing circuit 22 may further take into consideration programs assigned to other whole blood units when selecting a program for any particular whole blood unit. For example, processing circuit 22 may be configured to program units 0123 and 4416 for a plasma with red blood cell program based in part on processing circuit 22 already filling needed inventory (based on inventory data from source 36) for plasma by assigning a platelet with plasma program for unit 4230. As another example, processing circuit 22 may associate blood separator programs with donation identifiers based on data from the prioritization list, such as a prestored priority for a plasma with red blood cell program over a platelet with plasma program.

Processing circuit 22 may select programs for whole blood units individually or in batches (e.g., of 5, 10, 25 or more whole blood units) and may further be configured to store donation IDs of the whole blood units in association with programs selected for each donation ID. Processing circuit 22 may be configured to receive one of the donation IDs from a blood component separator 14 and, in response, transmit an identifier of one of the different blood separator programs (the one associated with the donation ID) to the blood component separator.

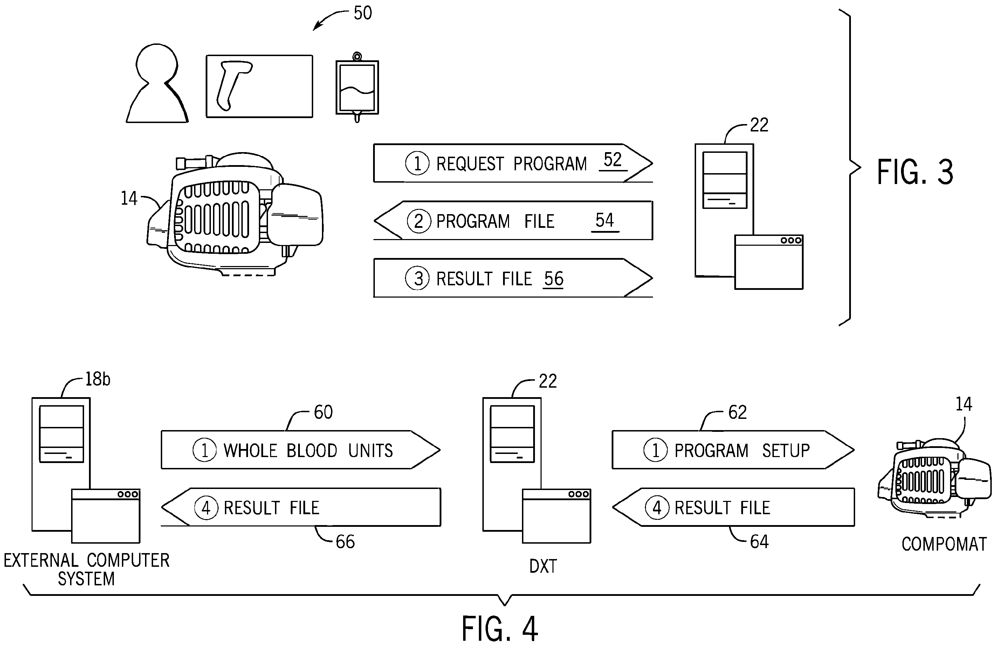

Referring now to FIGs. 3 and 4, methods of separating blood components from whole blood will be described according to illustrative embodiments. FIGs. 3 and 4 shows information record workflows or dataflows. As a preliminary step, whole blood units comprising whole blood donated and collected in primary containers or bags are each labeled with unique donation ID numbers. The labels may be implemented by machine-readable codes, such as barcodes, QR codes, etc. Donation ID codes may be used to distinguish a whole blood unit from other whole blood units for processing, supply chain management, order tracking, etc.

At item 50, a worker may use an electronic scanner to scan the donation ID from the whole blood container. The scanner may be a component of blood component separator 14 or a separate computing system disposed in the vicinity of separator 14. At block 52, separator 14 and/or scanning computer system may be configured to transmit a request message to processing circuit 22 (e.g., over one or more networks), the request message representing a request for a program or program identifier for running a blood component separation function. Processing circuit 22 may operate an algorithm, such as a manufacturing optimizer algorithm, to identify or select a blood separation program for execution by separator 14, which may be based on data from one or more of the data sources shown in FIG. 2, such as inventories 34, 36, target inventory 38, whole blood unit data 32, program identifiers 40, prioritization list 39, client device 18b, etc. At block 54, processing circuit 22 may be configured to transmit (e.g., over one or more networks) a program, program identifier, or file comprising multiple programs for the donation ID or multiple donation IDs to separator 14. Separator 14 may be programmed automatically based on the program, program ID, etc. and the separation may begin upon worker input from a worker on separator 14 confirming the program and/or desire to begin separation. A whole blood bag unit associated with the scanned ID is attached to separator 14, tubing is run through one or more pumps, presses, valves, etc., and output containers for separated blood components are provided to separator 14. After the separation is complete or stopped, at block 56 a result file or report may be transmitted from separator 14 to processing circuit 22 (or another computer) for storage, analysis, reporting, record-keeping, and/or other purposes. The result file may comprise one or more data about the separation procedure, such as duration of procedure, plasma volume, RBC volume, any error messages generated by separator 14, donation ID, donor characteristics, and/or other data.

Referring to FIG. 4, a client computer 18b external to processing circuit 22 may be used to implement parts of the workflow. At a block 60, client computer 18b may be configured to transmit a plurality of donation IDs for different whole blood units to processing circuit 22. Client computer 18b may receive the donation IDs from a BIS/BECS system, over a network from a blood donation center, on a USB drive, or via other methods. Processing circuit 22 may be configured to identify or select separation programs for each donation ID using one or more of the methods described herein. At block 62, a selected separation program for a particular donation ID may be transmitted over a network to blood component separator 14. After separation, a result file may be transmitted from separator 14 to processing circuit 22, as shown at block 64. Processing circuit 22 may be configured to store one or more result files in a database. Client computer 18b may further be configured to access result files for one or more separation procedures via block 66 by request from processing circuit 22.

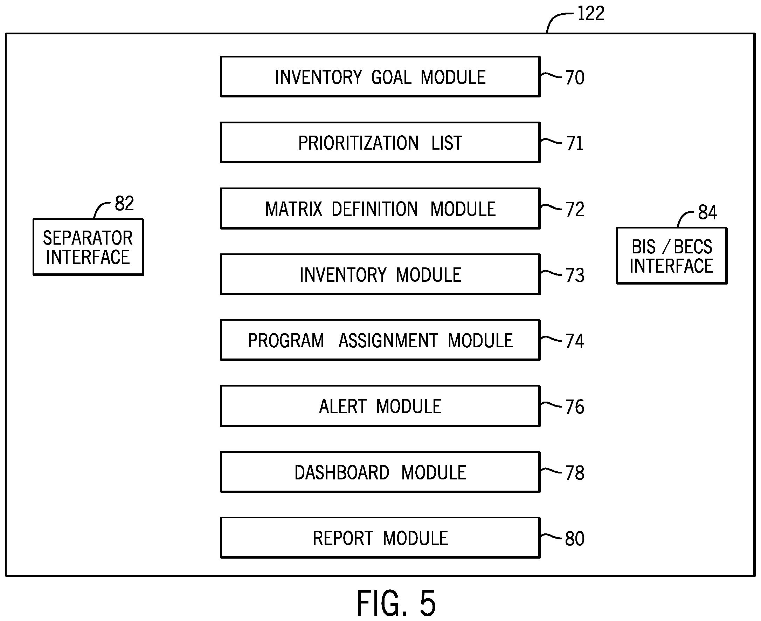

Referring now to FIG. 5, a processing circuit 122 is shown according to another illustrative embodiment. Features from processing circuit 22 and processing circuit 122 may be interchanged with one another, added or removed to achieve different embodiments. Processing circuit 122 may comprise one or more modules implemented as algorithms that may be applications, subroutines, or other code embodied on a tangible medium such as a memory circuit. An inventory goal module 70 may be configured to store inventory goals or targets for blood component products for a blood bank (e.g., target numbers/amounts of products of each type, such as A+ liquid plasma, O- FFP, etc.). Module 70 may be configured to store inventory goals for an entire organization comprising multiple manufacturing sites or for a particular manufacturing site. Inventory goals may comprise a number of products of each blood product type to be manufactured in a particular time frame. Module 70 may further be configured to store a number of products scheduled to be manufactured, such as those which are "work in progress," which may include those having programs sent to the separation devices already. Module 70 may further be configured to store a number of products to be manufactured, such as a total need. Inventory goal module 70 may be configurable by a worker having sufficient credentials or authorization level. The worker may be able to update inventory goals for one or more products at the site and/or organization level periodically, in real time, at any point in a day, once per day, or with other configurations. Processing circuit 122 may be configured to apply updated inventory goals to the selection of separation programs for donation IDs received after the update. In a case where donation IDs already have a separation program assigned and the inventory goal or goals are later updated, processing circuit 122 may further be configured to re-assign separation programs for such donation IDs.

Processing circuit 122 may further comprise a matrix definition module 72. Matrix definition module may be configured to receive a matrix definition from a worker, update the matrix definition, store the matrix definition, and/or serve responses to look up requests from processing circuit 122 or other computers. Matrix definition module 72 may store a matrix to associate separation program IDs with product types. For example, program ID 7 may indicate a cryo and cryo poor plasma separation program for AB- blood type. Program ID 4 may indicate a platelet and platelet poor plasma for A+ blood type. The matrix definition module 72 may in different embodiments also store rules for one or more of bag type, ABO Rh blood type, unit storage temperature, time-from-collection, and/or other data. Matrix definition module 72 may further be configured to receive a priority for one or more rules in the matrix. The priority may be a default priority, such as zero, for rules having the lowest priority. The priority may be one for higher priority and two for highest priority. Alternatively, a default priority may be a highest priority, with other priorities being associated with lower and lowest priorities. Priorities may have at least three different settings, at least five different settings, at least ten different settings, etc. Matrix definition module 72 may further be configured to be updated by a worker, such as a worker having a suitable authorization and/or credentials, which can be made by a worker from a client computer or other user interface periodically, in real time, at any point of day, once per day, or with other configurations.

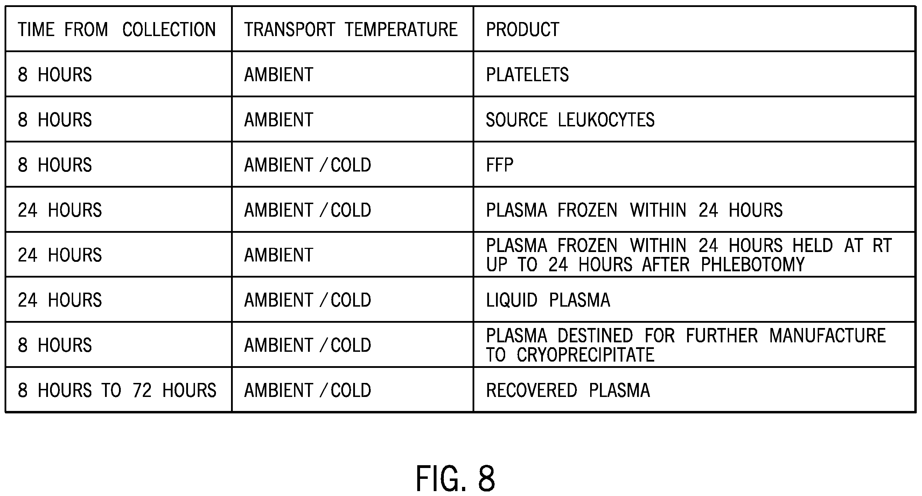

Referring to FIG. 8, an illustrative matrix is shown representing stored data elements in a matrix format. Each entry represents a product to be manufactured (e.g., platelets, source leukocytes, FFP, plasma frozen within 24 hours, plasma frozen within 24 hours held at room temperature up to 24 hours after phlebotomy, liquid plasma, plasma destined for further manufacture to cryoprecipitate, recovered plasma, etc.). Each product to be manufactured has one or more corresponding/associated characteristics such as the time from collection (e.g., 8 hours, 24 hours, 8 hours to 72 hours, etc.), corresponding/associated transport temperature (e.g., ambient, ambient or cold, etc.), and/or other characteristics such as ABO RH type, bag type, etc. The matrix may also be configured to store a worker-defined priority rule, such as high, medium, low, or other priority levels. As mentioned, processing circuit 122 may be configured to lookup a product or product available for collection based on one or more of the corresponding characteristics and base a selected program at least in part on data in the matrix.

Processing circuit 122 may further comprise an inventory module 73 comprising inventory data for labeled and/or non-labeled inventory, as described above. Inventory data may be updated continuously.

Processing circuit 122 may be configured to operate a program assignment module 74 configured to receive data from one or more data sources shown in FIG. 2 (as well as prioritization list module 71, inventory module 73, and/or inventory goal module 70), or other data sources, and to identify, select, or assign a blood separator program to each whole blood unit, by the unit's donation ID. The assignment module 74 may be configured to assign the blood separator program periodically, in real time, at any point of day, once per day, or with other configurations. The matrix definition module 72 may be configured to update the matrix periodically or repeatedly with updated data from one or more of the data sources, allowing program assignment module 74 to have access to current data from the data sources.

In some embodiments, program assignment module 74 is configured to receive a batch (e.g., at least 5, at least 10, at least 50, etc.) of donation IDs for whole blood units ready for manufacture/processing, from whole blood unit data source 32. Program assignment module 74 may be configured to assign separation programs to each donation ID and store the IDs in a memory. In response to a later request from a blood component separator 14 comprising donation ID, received via a separator interface 82 (e.g., network interface circuit in communication with separator 14), processing circuit 122 recalls from memory the previously stored program for the donation ID and transmits a response message with the program ID. In another embodiment, program assignment module 74 does not assign a program ID to a donation ID until a request is received from blood component separator 14 via separator interface 82 for a program ID. Processing circuit 122 may be configured to determine which program is to be used at the time of the request from blood component separator 14. In another embodiment, program assignment module 74 may store a batch of donation IDs associated with selected separator programs and, if a donation ID is received with a request for a program that is not in the batch, module 74 then selects a separator program based on one or more of the data sources and/or matrix data derived therefrom. In another embodiment, program assignment module 74 may store a batch of donation IDs associated with selected separator programs and, if a donation ID is received with a request for a program that is not in the batch of whole blood unit data 32 or whole blood components that are "work in progress," then the separator program may generate an error requesting the donation ID to be added to the system.

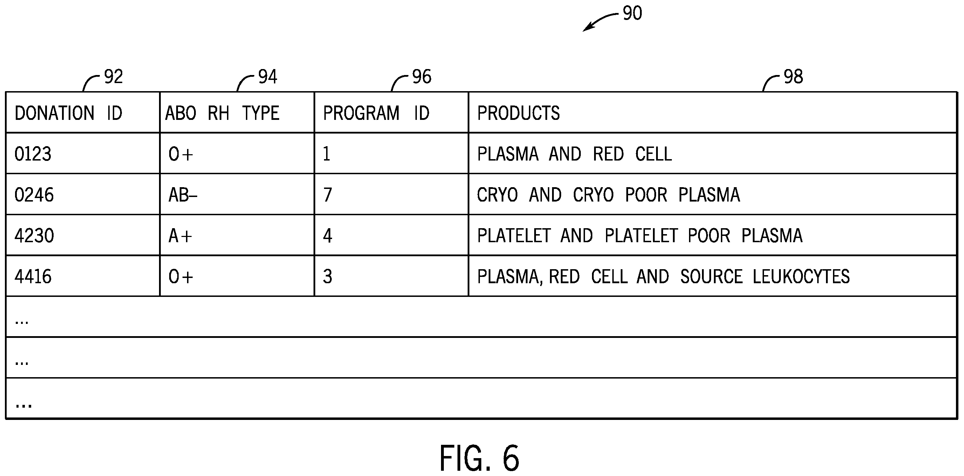

FIG. 6 shows a sample display screen 90 according to an illustrative embodiment. Display screen 90 may be an overview screen or dashboard screen and may be generated by dashboard module 78 automatically and/or by request from a worker from a client device, blood separator having a user interface, or other terminal in communication with processing circuit 122. In this embodiment, display screen 90 displays all or some whole blood units to be processed. A plurality of fields may be provided to show data associated with the whole blood unit, such as a donation ID field 92, a blood type field showing blood type of the whole blood unit 94, a program ID field 96, product field 98, and/or other fields. Product field 98 may be a textual description of the separation program--or products produced by said program--to be run on the whole blood unit having the donation ID. Products may be plasma and red cell products, cryo and cryo poor plasma products, etc. in number of units, in milliliters and/or grams, and/or other data regarding products to be manufactured. An additional data field may comprise a facility ID and/or organization ID to allow a worker to filter donation IDs by facility (manufacturing site) and/or organization.

In some embodiments, display screen 90 may be configured to receive a manual selection from a worker of a separation program via a user interface. The manual selection may override the program ID and/or products to be collected, or the manual selection may input a program ID and/or products to be collected for a donation ID not having a program assignment. The manual selection may be stored in processing circuit 122 and sent to a blood component separator in response to a request for a program for the donation ID associated with the manual selection.

In another embodiment, the processing circuit may be configured to generate a display screen showing status to goals, work in progress and/or manufacturing statistics. Two or more of these objects may be shown simultaneously on a display screen. Status to goals may be shown, for example, as a percentage for each type of blood product. Work in progress may illustrate a number of blood products being processed or processed in that day. Other manufacturing statistics may include productivity data, locations, time to process, etc.

Referring again to FIG. 5, processing circuit 122 may be configured to support two-way interfaces with other devices, such as a plurality of separators via separator interface 82, one or more BIS/BECS systems via BIS/BECS interface 84, and/or interfaces to other data sources shown in FIG. 2 or otherwise provided. Interfaces may alternatively be one-way. In one example interface, BIS/BECS interface 84 may be configured to receive a file containing donation IDs for whole blood units collected for an hour, day or since a last download of such a file. Interface 84 may be configured to allow download/receipt of such a file at any point during a day. In another example interface, separator interface 82 may be configured to receive a donation ID from a particular separator, and processing circuit 122 may assign a separator program and transmit the program ID to the particular separator for the received donation ID. Processing circuit 122 may transmit the program ID instantly, within less than 30 seconds, less than a minute, etc., from when the donation ID is received.

A report module 80 of processing circuit 122 may be configured to process results of a separation. In one example, report module 80 may be configured to receive a result file from a blood component separator via separator interface 82 and update an aggregated statistical dashboard or other database. Report module 80 may also be configured to receive an electronic report created by a worker after performing a manual separation. In some embodiments, report module 80 may be configured to send the result file or result data therefrom to a BIS/BECS via BIS/BECS interface 84 to update an inventory file and/or other data stored in the BIS/BECS. A result file or report from a blood component separator may comprise an indication of the blood separator program used and may comprise an indication that the worker who programmed the blood component separator overrode or did not accept the program assigned by processing circuit 122 but instead selected a different program. In another example, report module 80 may be configured to compile result data from a plurality of blood component separators over a period of time (e.g., an hour, a day, a week, etc.) and generate a report or alert indicating number of units or percentage of units overridden by a worker (e.g., the worker did not follow or accept the program selected by the program assignment module 74) but instead manually programmed the separator. Processing circuit 122 may further comprise an alert module 76 configured to generate and transmit alerts to preprogrammed recipients (e.g., by email address, mobile phone number, etc.) based on the occurrence of certain events, such as a percentage of overridden separations by a particular worker or facility location, etc. Such data can be used to improve rules, weights, or other data of the matrix definition module 72 and/or program assignment module 74. Alert module 76 may be customizable to indicate rules triggering alerts, recipients to receive alerts, frequency of alerts, etc. Alert module 76 may be configured to feed alerts to a dashboard or overview screen, or other display screen, showing for example when whole blood units are ready to be processed/manufactured, when a certain number of units exceeding a threshold are ready, when units older than predetermined age are ready for processing, and/or other alerts.

Report module 80 may further be configured to generate display screen 90 or other display screens based on result data from blood component separators which are not optimized using processing circuit 122. For example, a blood component separator may be used during a network outage or at a facility that does not have communication with processing circuit 122. Result data from such blood component separators may be manually entered into BIS/BECS and then retrieved by report module 80 and reported on a dashboard screen or other display screen. Results such as amount of blood product of each type manufactured, blood type, donation ID, worker ID, duration of separation, and/or other data may be retrieved and reported in this manner by report module 80. In some embodiments, report module may implement a time feature that automatically concludes a whole blood unit has been manually processed after a predetermined period of time, e.g., X hours.

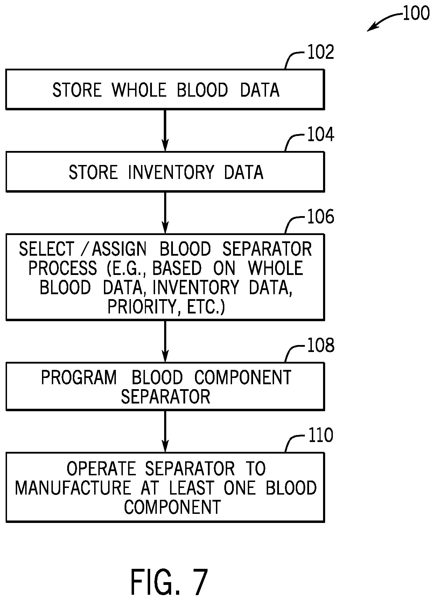

FIG. 7 is a flowchart of a method 100 of separating whole blood into blood component products, according to an illustrative embodiment. The method may include one or more of these steps, and/or one or more of the features described hereinabove, in different embodiments. At block 102, the method may comprise storing whole blood characteristic data and blood center inventory data in a memory circuit. Whole blood characteristics may comprise one or more of a unique donation ID for each whole blood unit, date and time the unit was collected, bag type, whether transported on ice or not, weight or volume, and/or other data. Whole blood characteristic data may be automatically generated and stored in a database, for example by way of a barcode scanner, collection device report, etc., or may be manually entered into the database, or may comprise data elements both automatically generated and manually entered or annotated. At block 104, the method may comprise storing blood center inventory data, such as the numbers of different types of blood products (e.g., AB+ platelets, O- plasma, RBC, etc.). The inventory data may be for one facility, a plurality of facilities, or for an organization of multiple facilities. The blood center may be a blood collection facility, blood processing facility, or other organization involved in any step of a supply chain of blood or blood products.

At a block 106, the method may comprise using the whole blood characteristic data and blood center inventory data to select a blood separator program. In one example, block 106 may select a cryo and cryo poor plasma program for a particular donation ID and based on an indication of low inventory for cryo and cryo poor plasma (e.g., inventory below a predetermined threshold, which may be customizable by a worker). In another example, the blood center inventory data may comprise a number of platelet and platelet poor plasma products in inventory at the blood center and/or an indication that a number of platelet and platelet poor plasma products is less than a predetermined number of platelet and platelet poor plasma products. Method block 106 may select or assign a blood separator program based on the indication that the number of plasma products is less than the predetermined number of plasma products. The blood separator program may comprise a plasma product collection.

At a block 108, the method may comprise programming a blood component separator with the selected blood separator program. The program may be prestored in the blood component separator, for example in firmware or other algorithms embodied on a tangible medium such as a memory circuit. The program may alternatively be downloaded to the selected blood separator program after the selection in block 106. In some examples, a worker uses the selected blood separator program following the scan of the blood component identifier. In some examples, the blood separator may prompt the worker to confirm the use of the selected blood separator program. In some examples, the blood separator program may be configured to allow the worker to override or otherwise select a blood separator program different than the one selected in block 106.

At a block 110, the method may comprise operating the selected blood separator program to receive whole blood from a container and to separate at least one blood component from the whole blood for collection in a blood component container. In some embodiments, at least two, or at least three blood components are separated from the whole blood in the container. The whole blood may be a blood donation collected at a different location than the laboratory location of the blood component container in which the worker operates. The whole blood unit may have been stored for a period of time prior to processing by the blood component separator. The whole blood container may be attached to the blood components separator, with tubing being placed near suitable clamps, pumps, presses, sensors, and/or other features of the separator. The separator may be configured to sense and confirm proper placement of the whole blood container on the separator before allowing the technician to begin the separation. One or more collection bags may be coupled to the separator to process separated blood components, such as platelets, plasma, red blood cells, etc.

Block 106 may be preceded by additional method blocks, such as scanning a donation identifier from the whole blood container. The scanning may comprise using a machine-readable indicator on the whole blood container and a scanner coupled to or used with the blood component separator. The separator and/or scanner may then transmit the donation identifier from the blood component separator to a remote processing circuit, such as processing circuit 22, 122. The separator may then receive from the remote processing circuit the selected blood separator program for the scanned donation identifier.

Other method blocks are contemplated, such as receiving an inventory goal from a client computer and selecting the blood separator program based further on the inventory goal. For example, the inventory goal may comprise a predetermined number or amount of blood component product of an ABO Rh blood type or other blood characteristics.

In some embodiments, processing circuit 22, 122 may comprise a forecasting engine. At the conclusion of manufacturing one or a plurality of blood component products, the forecasting engine may use current inventory levels and hospital blood component demand to forecast collection needs for a worker-defined period of time (e.g., day, week, month). For example, if a customer places a large order for a cryo component, then forecasting engine would update circuit 22 with an updated product need (target inventory 38) and/or prioritization list 39. Hospital blood components data sources may be provided as an additional data source to those described with reference to FIG. 2.

The various functionality described herein may be implemented by a system comprising one or more computers, such as one or more servers coupled via a communication network, one or more blood component separator devices, and one or more worker workstations.

Computing systems and processing circuits described herein may include a processor, such as a general-purpose processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. The processor may also be implemented as a combination of computing devices, e.g., a combination of a DSP and a microprocessor, a plurality of microprocessors, one or more microprocessors in conjunction with a DSP core, or any other such configuration. Computing systems and processing circuits described herein may comprise memory circuits configured to store data and instructions. The memory may be a storage device coupled to a processing circuit and/or may be integral to the processing circuit. The memory may store a set of instructions, in the form of software code, which a processing circuit is configured to execute. Additionally, the memory may be configured to store data received from remote devices via the communication interface 350. The storage devices can also include a disk drive, for example, a hard disk drive. Other volatile or non-volatile storage devices may additionally or alternatively be used, including optical discs, floppy discs, magnetic tape, and Zip drives.

Computing systems and processing circuits described herein may comprise a communication interface configured to receive information from, and/or transmit information to, remote devices.

Processing circuits and computers described herein may operate with software stored in memory and may execute an operating system, such as, for example, a Windows, UNIX, Mac OS, or Solaris operating system. Processing circuits and computers may also execute software applications stored in the memory. The software applications may include code written in any suitable programming language known to those skilled in the art, including, for example, Java and C++ programming languages. In some embodiments, memory may include software for operating the computing system as a web server.

Computing systems and processing circuits described herein may include or be coupled to one or more input devices and/or output devices. Such devices may enable a system worker to enter inputs into, and receive outputs from, the system. Input devices may include, for example, a keyboard, mouse, touchscreen, button, switch, RFID reader, Bluetooth circuit, and/or microphone, and output devices may include, for example, a display, printer, or speakers.

In the above detailed description, reference is made to the accompanying drawings, which form part of the present disclosure. The embodiments described in the drawings and description are intended to be exemplary and not limiting. Other embodiments may be utilized, and modifications may be made without departing from the spirit or the scope of the subject matter presented herein. Aspects of the disclosure, as described and illustrated herein, can be arranged, combined, and designed in a variety of different configurations, all of which are contemplated and form part of this disclosure. The term "comprising" or "comprises" is intended to mean that the devices, systems, and methods include the recited elements, and may additionally include any other elements. The singular form "a," "an" or "the" include both singular and plural elements. For example, and without limitation, "a blood component" includes one or more blood components, and "a donor" may refer to one or a plurality of donors.

## Claims

1. A system for optimizing the separation of whole blood into blood components, comprising:
a processing circuit configured to receive whole blood characteristic data and inventory data and to identify a whole blood separation program based on the whole blood characteristic data and the inventory data; and
a blood component separator configured to receive a container comprising whole blood and to operate the identified blood separator program to separate at least one blood component from the whole blood for collection in a blood component container.

2. The system of Claim 1, wherein the processing circuit is configured to identify the blood separator program based on the inventory data indication that a number of blood products is less than a predetermined number of blood products.

3. The system of any one of Claims 1 through 2, wherein the whole blood characteristic data comprises a donation characteristic of the whole blood, the donation characteristic comprising one or more of donor ABO Rh blood type, donor gender, donor antibodies, donor hgb or hct, donor pregnancy history, collection time, collection duration, product type, transportation temperature, storage temperature.

4. The system of Claim 3, wherein the processing circuit is configured to identify the blood separator program based on an indication that a number of blood component products of the ABO Rh blood type in the inventory data is less than a predetermined number of blood component products of the ABO Rh blood type.

5. The system of any one of Claims 1 through 4, wherein the blood component separator is configured to transmit a request message to the processing circuit, wherein the processing circuit is configured to transmit a response message comprising the identity of the blood separator program.

6. The system of any one of Claims 1 through 5, further comprising a client computer configured to receive an inventory goal from a worker and to transmit the inventory goal to the processing circuit, wherein the processing circuit is configured to identify the blood separator program based further on the inventory goal.

7. The system of any one of Claims 1 through 6, wherein the whole blood characteristic data is received from a blood bank information system dedicated to a blood bank, wherein the whole blood characteristic data comprises donation identifiers and ABO Rh blood type for a plurality of whole blood products.

8. The system of any one of Claims 1 through 7, wherein the processing circuit is configured to generate a display screen showing each of a plurality of donation identifiers and associated different blood separator programs identified by the processing circuit.

9. The system of any one of Claims 1 through 8, further comprising a client computer configured to receive a blood product type priority from a worker and to transmit the blood product type priority to the processing circuit, wherein the processing circuit is configured to identify the blood separator program based further on blood product type priority.

10. The system of any one of Claims 1 through 9, wherein the processing circuit is configured to generate a display screen showing status to goals, work in progress and/or manufacturing statistics.

11. A method of separating whole blood into blood components, comprising:
storing whole blood characteristic data and at least one of inventory data and a prioritization list in a memory circuit;
selecting a blood separator process with a processing circuit based on the whole blood characteristic data and the at least one of inventory data and the prioritization list; and
using the selected blood separator process to separate at least one blood component from whole blood in a first container for collection in a second container or multiple containers.

12. The method of Claim 11, further comprising:
programming a blood component separator with the selected blood separator program;
scanning a donation identifier from the first container;
transmitting the donation identifier from the blood component separator to a remote processing circuit; and
receiving from the remote processing circuit the selected blood separator process for the scanned donation identifier.

13. The method of any one of Claims 11 through 12, further comprising selecting the blood separator process based on an indication that a number of blood components is less than a predetermined number of blood components, wherein the selected blood separator process comprises a blood components process, wherein the at least one blood component which is collected in the blood component container.

14. The method of any one of Claims 11 through 13, further receiving an inventory goal from a client computer, the processing circuit selecting the blood separator process based further on the inventory goal.

15. The method of Claim 14, wherein the inventory goal comprises a predetermined number or amount of blood component product of an ABO Rh blood type.
